# EUROPEAN PATENT SPECIFICATION

(11) **EP 1 780 216 B1**
(45) Date of publication and mention of the grant of the patent: **29.12.2010**
(21) Application number: 05023371.7
(22) Date of filing: 26.10.2005
(51) Int. Cl.: C07K 14/165, C12N 15/50, C12N 7/06, C12N 7/08, C07K 16/10, A61K 39/215, A61K 48/00, G01N 33/53

(54) **Pantropic canine coronavirus**
Pantropisches Hundecoronavirus
Coronavirus canin pantropique

(43) Date of publication of application: 02.05.2007
(60) Divisional application: 10176280.5
(73) Proprietor: Buonavoglia, Canio, 70016 Noicattaro (BA) (IT)
(72) Inventor: Buonavoglia, Canio, St. per Casamassima Km 3 70010 Valenzano (IT); Decaro, Nicola, St. per Casamassima Km 3 70010 Valenzano (IT); Martella, Vito, St. per Casamassima Km 3 70010 Valenzano (IT); Elia, Gabriella, St. per Casamassima Km 3 70010 Valenzano (IT); Campolo, Marco, St. per Casamassima Km 3 70010 Valenzano (IT); Desario, Costantina, St. per Casamassima Km 3 70010 Valenzano (IT); Tempesta, Maria, St. per Casamassima Km 3 70010 Valenzano (IT)
(74) Representative: Banfi, Paolo

(56) References cited:
- EP-A- 0 510 773
- EP-A2- 0 329 264
- WO-A1-2004/011651
- US-A- 4 904 468
- HORSBURGH B C ET AL: "ANALYSIS OF A 9.6 KB SEQUENCE FROM THE 3' END OF CANINE CORONAVIRUS GENOMIC RNA" JOURNAL OF GENERAL VIROLOGY, SOCIETY FOR GENERAL MICROBIOLOGY, SPENCERS WOOD, GB, vol. 73, January 1992 (1992-01), pages 2849-2862, XP002015843 ISSN: 0022-1317
- DATABASE UniProt [Online] 1 November 1996 (1996-11-01), "ORF 3X." XP002374208 retrieved from EBI accession no. UNIPROT:Q65983 Database accession no. Q65983
- VENNEMA H ET AL: "Genomic organization and expression of the 3' end of the canine and feline enteric coronaviruses." VIROLOGY. NOV 1992, vol. 191, no. 1, November 1992 (1992-11), pages 134-140, XP009064213 ISSN: 0042-6822
- DATABASE UniProt [Online] 1 February 1994 (1994-02-01), "Nonstructural protein 7 (11 kDa protein) (X3 protein) (6A protein)." XP002374209 retrieved from EBI accession no. UNIPROT:Q04703 Database accession no. Q04703
- PRATELLI ANNAMARIA ET AL: "Two genotypes of canine coronavirus simultaneously detected in the fecal samples of dogs with diarrhea." JOURNAL OF CLINICAL MICROBIOLOGY. APR 2004, vol. 42, no. 4, April 2004 (2004-04), pages 1797-1799, XP009064215 ISSN: 0095-1137
- SANCHEZ-MORGADO ET AL: "Molecular characterization of a virulent canine coronavirus BGF strain" VIRUS RESEARCH, AMSTERDAM, NL, vol. 104, no. 1, August 2004 (2004-08), pages 27-31, XP005107900 ISSN: 0168-1702
- DECARO ET AL: "Genotype-specific fluorogenic RT-PCR assays for the detection and quantitation of canine coronavirus type I and type II RNA in faecal samples of dogs" JOURNAL OF VIROLOGICAL METHODS, AMSTERDAM, NL, vol. 130, no. 1-2, December 2005 (2005-12), pages 72-78, XP005150561 ISSN: 0166-0934 [retrieved on 2005-07-15]
- C. BUONAVOGLIA, N. DECARO, V. MARTELLA, G. ELIA, M. CAMPOLO, C. DESARIO, M. CASTAGNARO AND M. TEMPESTA: "Canine Coronavirus Highly Pathogenic for Dogs" EMERGING INFECTIOUS DISEASES, [Online] vol. 12, no. 3, March 2006 (2006-03), pages 492-494, XP002374370 Atlanta, USA ISSN: 1080-6059 Retrieved from the Internet: URL:http://www.cdc.gov/ncidod/EID/vol12no0 3/pdfs/05-0839.pdf> [retrieved on 2006-03-27]

### SEQUENCE LISTING

<110> Buonavoglia, Canio
<120> pantropic canine coronavirus
<130> 1577EUR
<160> 19
<170> PatentIn version 3.3
<210> 1
   <211> 8745
   <212> DNA
   <213> Unknown
<220>
   <223> canine coronavirus
<400> 1
   ggtaagttgc tcattagaaa taatggcaag ctactaaact ttggtaatca tttagttaat      60
   gtgccatgat tgtgatctta acttgcgtct tattgttgtg ctcgtaccat actgttgcga     120
   gtacgacaaa taatgattgt agacaagtta acgtaacaca attagatggc aacgaaaatc     180
   tcattagaga ctttttgttt caaaacttta aagaagaagg aactgtagtt gttggtggct     240
   attatcctac agaggtgtgg tacaactgtt ctagaacagc acctaccact gcctatgagt     300
   attttaataa catacatgca ttttattttg atatggaagc catggaaaat agcactggta     360
   atgcacgtgg taaaccgtta ttgtttcatg ttcatggtga gcctgttagt gctatcatat     420
   atatatcggc ttatagaaat gatgtacaac acaggccact tttaaaacat gggttagtgt     480
   gcataactaa aactcgcaat attgactata acagcttcac tagcagacag tggaattcca     540
   tatgtacagg taatgacaga aaaattcctt tctctgtcat acccacggac aatggaacaa     600
   aaatctatgg acttgagtgg aatgacgaat ttgttacagc ctatattagt ggtcattctt     660
   ataattggaa catcaataat aattggttta acaatgttac actactttat tcccgctcaa     720
   gtactgctac atggcaacac agtgctgcat atgtttacca aggtgtttct aacttcactt     780
   attacaagtt aaataacacc aatggtttaa aaacttatga attttgtgat gattatgaat     840
   attgcactgg ctacgctact aatgtctttg ctcccactgt aggaggttac atacctgatg     900
   gatttagttt taacaattgg tttttgctta caaatggctc cacttttgtt agtggcagat     960
   ttgtaacaaa tcaaccatta ttagttaatt gtttatggcc agtgcccagt tttggtgtgg    1020
   cagcacaaga attttgtttt gaaggtgcgc agtttagtca gtgcaatggt gtgtctttaa    1080
   ataacacagt ggatgttatt agattcaatc ttaattttac cgcagatgta caatctggca    1140
   tgggtgctac agtgttctca ttgaatacaa caggtggtgt cattcttgaa atttcatgtt    1200
   ataatgacac agtgagtgag tctagttttt acagttatgg tgaaattcca tttggcataa 1260
   ctgatggacc acggtactgt tatgcacttt acaatggcac agctcttaag tatttaggaa 1320
   cattaccacc tagtgtaaag gaaattgcta ttagtaagtg gggccatttt tatattaatg 1380
   gttacaattt ctttagcaca tttcctattg attgtatatc ttttaattta accactggta 1440
   ctagtggagc tttttggact attgcttaca catcgtacac tgaagcatta gtacaagttg 1500
   aaaacacagc tattaaaaag gtgacgtatt gtaacagtca cattaataac attaaatgtt 1560
   ctcaacttac tgctaatttg caaaatggtt tttatcctgt tgcttcaagt gaagttggtc 1620
   ttgtcaataa gagtgttgtg ttactaccta gtttctattc acataccagt gttaatataa 1680
   ctattgatct tggtatgaag cgtagtggtt atggtcaacc tatagcctca gcattaagta 1740
   acatcacact accgatgcag gataataaca ccgatgtgta ttgcattcgt tctaatcaat 1800
   tttcagttta cgtgcattcc acttgtaaaa gttctttatg ggacaatgtg tttaattctg 1860
   actgcacaga ttttttacat gctacagctg ttataaaaac tggtacttgt cctttctcat 1920
   ttgataaatt gaacaattac ctaactttta acaagttctg tttttcattg aatcctgttg 1980
   gtgccaattg caaatttgat gttgctgccc gtacaagaac caatgagcag gttgttagaa 2040
   gtttatatgt gatatatgaa gaaggagaca atatagtggg tgtaccgtct gataatagtg 2100
   gtctgcacga tttgtcagtg ttacacttag actcctgtac agattacaat atatatggta 2160
   gaactggtgt tggtattatt agacaaacta acagcacact acttagtggc ttatattaca 2220
   catcactatc aggtgatttg ttaggtttta aaaatgtcac tgacggtgtt gtctattctg 2280
   taacaccatg tgatgtaagt gcacaagctg ctgttattga tggtgccata gttggagcta 2340
   tgacttccat taatagtgaa ctgttaggtc taactcattg gacaacaaca cctaattttt 2400
   attattactc catatataat tatacaaatg cgagaactcg tggcactgca atcgacagta 2460
   acgatgttga ttgtgaacct atcataacct attctaatat aggtgtttgt aaaaatggag 2520
   ctttggtttt tattaacgtc acacattctg atggtgacgt tcaaccaatt agcaccggta 2580
   atgtcacgat acctacaaat tttactatat ctgtgcaagt cgaatatatt caggtttaca 2640
   ctacaccagt gtcaatagac tgttcaagat acgtctgcaa tggtaatcct agatgcaata 2700
   aattgttaac acaatatgtc tctgcatgtc aaactattga gcaagcactt gcaatgggtg 2760
   ccagacttga aaacatggaa gttgattcca tgttgtttgt ttctgaaaat gcccttaaat 2820
   tggcatctgt tgaagcattc aatagtacgg aaactctaga tcctatttac aaagaatggc 2880
   ctaacattgg tggttcttgg ctaggaggtt taaaagacat attgccatct cacaatagca 2940
   aacgtaagta ccggtctgct atagaagatt tgctttttga taaggttgtg acatctggct 3000
   taggtacagt cgatgaagat tacaaacgtt gtacgggtgg ctatgacata gctgacttag 3060
   tgtgtgcaca atattacaat ggcatcatgg tgttacctgg tgtggctaat gatgacaaga 3120
   tggctatgta cactgcatct cttgcaggtg gtataacatt aggtgcactt ggtggtggtg 3180
   cagtgtctat accttttgca gtagcagttc aggctagact taattatgta gctttacaaa 3240
   ctgacgtatt gaacaaaaac cagcagattc tggctaatgc tttcaatcaa gcgattggta 3300
   acattacaca ggcatttggt aaggttaatg atgctataca tcaaacgtca aaaggtcttg 3360
   ctactgttgc taaagcattg gcaaaagtgc aagatgttgt taacacacaa gggcaagctt 3420
   taagccacct aacagtacaa ttgcaaaata attttcaagc cattagtagt tccattagtg 3480
   acatttataa caggcttgat gagttgagtg cggatgcaca agttgacagg ctgattacag 3540
   gacgacttac agcacttaat gcatttgtgt ctcagacttt aaccagacaa gcagaggtta 3600
   gggctagtag acaacttgct aaagacaaag ttaatgaatg cgttaggtct caatcccaaa 3660
   gatttggatt ctgtggtaat ggtacacatt tgttttcact tgcaaatgca gcaccaaatg 3720
   gcatgatttt ctttcacaca gtgctattac caacagctta tgaaactgtg acggcctggt 3780
   caggtatttg tgcatcagat ggcgatcgca cttttggact tgttgttaaa gatgtccagc 3840
   tgacgctatt tcgcaattta gatgacaaat tctatttgac acctagaact atgtatcagc 3900
   ctagagttgc aactagttct gattttgttc aaattgaagg gtgtgatgtc ttgtttgtca 3960
   atgcaactgt aattgagttg cctagtatta tacctgatta tatcgatatt aatcagactg 4020
   ttcaagacat attagaaaat tacagaccaa attggacagt acctgaatta acacttgaca 4080
   ttttcaacgc aacctactta aacctgactg gtgaaattaa tgacttagaa ttcaggtcag 4140
   aaaagctaca taacaccacg gtagaacttg ctgttctcat tgacaatatt aacaatacat 4200
   tagtcaatct tgaatggctc aatagaattg aaacttatgt aaaatggcct tggtatgtat 4260
   ggctactaat aggcttagta gtaatatttt gcataccatt actgctattt tgctgttgta 4320
   gtacaggttg ctgtgggtgc ataggttgct taggaagttg ttgtcactct atttgtagta 4380
   gaagacaatt tgaaaattat gaaccaattg agaaagtgca tgtccattaa attcaaaata 4440
   aatctcttaa gaactaaact tatgagtcat tacaggtctt gtatggacat tgtcaaatct 4500
   attgacacat ccgtagacgc tgtacttgac gaacttgatc gtgcatactt tgctgtaact 4560
   cttaaagtag agtttaagac tggtaaacta cttgtgtgta taggttttgg tgatacacct 4620
   cttgaggcta aggataaagc atatgctaaa cttggtttct ctattattga agaagtcaat 4680
   agtcatacag ttgtttgata ttaccttttg aaactagact tcttatcatc gaacaaacaa 4740
   aacctaaagc attaagtgct acaaaacaat caaagagaga ttatagaaaa attgccattc 4800
   taaattccat gagaaaatga ttggtggact ttttcttaac actctgagtt ttgtaattgt 4860
   tagcaaccat gtcattgtta ataatacagc aaatgtgcat tctatacaac aagaacatgt 4920
   tatagtacaa cagcatcagg ttgttagtgc tagaacacaa aattactacc cagagttcag 4980
   catcgctgta ctctttgtat tatttctagc tttgtaccgt agtacaaact ttaagacgtg 5040
   tgtcgatatc ttaatgttta agattgtatc aatgacactt atagggccta tgcttatagc 5100
   atatggttac tacattgatg gcattgttac aacaactgtc ttagctttaa gatttgtcta 5160
   cttatcatac ttttggtatg ttaatagtag gtttgaattc atcttataca atacaacgac 5220
   actcatgttt gtacatggca gagctgcacc gtttatgaga agttctcaca gctctattta 5280
   tgtcacattg tacggtggca taaattatat gtttgtgaat gacctcacgt tgcattttgt 5340
   agaccctatg cttgtaagca tagcaatacg tggcttagct catgctgatc taactgttgt 5400
   tagagcagtt gaacttctca atggtgattt tatctatgta ttttcacagg agcccgtagt 5460
   cggtgtttac aatgcagcct tttctcaggc ggttctaaac gaaattgact taaaagaaga 5520
   agaagaagac catacctatg acgttcccta gggcattgac tgtcatagat gacaatggaa 5580
   tggtcattag tatcattttc tggttcctgt tgataattat attgatatta ctttcaatag 5640
   cattgctaaa tataattaag ctatgcatgg tatgttgtaa cttaggaaga acagttatta 5700
   ttgttccagc gcaacatgcc tatgatgcct ataagaattt tatgcgaatt aaagcatata 5760
   accccgacga agcactcctt gtttgaacta aacaaaatga agattttgtt agtattagcg 5820
   tgtgcaattg catgcgcatg tggtgaacgt tattgcgcta tgaaatctga ctcagatact 5880
   tcgtgtcgca atggtaccac tactgattgc gaatcatgct tcaacggagg tgatcttatt 5940
   tggtatcttg caaactggaa cttcagctgg tctgtaatat tgatcgtttt tataacggtt 6000
   ttacaatatg gtagacctca atttagctgg ttcgtgtatg gcattaaaat gcttattatg 6060
   tggctactat ggcccattgt tctggctctt acgattttta atgcatactc ggaatacgaa 6120
   gtttccagat atgtaatgtt cggctttagt gttgcaggtg caattgttac atttatactt 6180
   tggattatgt attttgttag atccattcag ttatacagaa ggactaagtc ttggtggtct 6240
   ttcaaccctg aaactaacgc aattctttgc gttagtgcat taggaaggag ctatgtgctt 6300
   cctcttgaag gtgtgccaac tggtgtcact ttaacattgc tctcagggaa tttgtacgct 6360
   gaagggttca aaattgcagg tggtatgaac atcgacaatt tgccaaagta cgtaatggtt 6420
   gcattaccta gcaggaccat tgtctacaca cttgttggta agaaattgaa agcaagtagt 6480
   gcaacaggat gggcttacta tgtaaaatct aaagctggtg attactcaac agatgcacga 6540
   actgacaatt tgagtgagca agaaaaatta ttacatatgg tataactaaa cttctaaatg 6600
   gccaaccagg gacaacgcgt tagctgggga gatgaatcta ccaaaaagcg tggtcgttcc 6660
   aattctcgtg gccggaagaa taatactata cctctttcat tcttcaaccc cattaccctc 6720
   caacaaggtt caaaattttg gaacttatgt ccgagagact ttgtacccaa aggaataggt 6780
   aacaaggatc aacagattgg ttattggaat agacaaactc gctatcgcat ggtgaagggt 6840
   cagcgtaaag agcttcctga aaggtggttc ttctactatt taggtactgg tcctcacgcc 6900
   gatgctaaat ttaaagatag aatagatgga gttgtctggg ttgccaagga tggtgccatg 6960
   aataagccaa ccacacttgg taatcgtggt gctaataatg aatccaaagc tttgaaattc 7020
   gatggtaaag taccagtaga atttcaactt gaagtgaacc aatcaaggga caattcaagg 7080
   tcacgctctc aatctagatc ccagtctaga aatagatctc aatctagagg aaggcaacaa 7140
   tccaataaca agaaggatga cagtgtagaa caagctgttc ttgctgcact caaaaagtta 7200
   ggtgttgaca cagaaaaaca acaacaacgc tctcgttcta aatccaagga acgtagcaac 7260
   tctaagacaa gagacactac acctaagaat gaaaacaaac acacctggaa gagaactgca 7320
   ggtaaaggtg atgtgacaaa attttatgga gctagaagta gttcagccaa ttttggtgac 7380
   agcgatctcg ttgccaatgg tagcggtgcc aagcattacc cacaactggc tgaatgtgtt 7440
   ccatctgtat ctagcattct gtttggaagc tattggactg caaaggaaga tggcgaccag 7500
   attgaagtca cattcacaca taaataccac ttgccaaaag atgatcctaa gactggacaa 7560
   ttccttcagc agattaatgc ctatgctcgt ccatcagagg tggcaaaaga acagagacaa 7620
   cgcaaagctc gttctaaatc tgcagaaagg tcagagcaag aggttgtacc tgatgcatta 7680
   acagaaaatt acacagatgt gtttgatgac acacaggttg agattattga tgaggtaacg 7740
   aactaaacga atgctcgttt tcctccatgc tgtatttatc acagttttaa tcttactact 7800
   aattggtaga ctccaattat tagaaagatt attacttaat cattccctca atcttaaaac 7860
   tgtcaataat gttttaggtg tgactgacac tggtctgaaa gtaaattgct tacagctttt 7920
   gaaaccagac tgtcttgatt ttaacatctt acataggagt ttggcagaaa ccagattact 7980
   aaaagtagta cttcgagtaa tctttctagt attactaggg ttttgctgct acagattgtt 8040
   agtcacatta atttagcatc atgaagtttt tgatttttgt actgtgtctt tctcttgtga 8100
   atggatatgg aattagaaga agcatacaag aacatgaccc aaaagagtcc catgaacacc 8160
   cgaccatgac atgggaatta ttggaaagat ttgttggaag taccttgtac atcacaacaa 8220
   accaaatcct atctctacca actggagcgc aaatttattg tgatgagatt gaaggattcc 8280
   aatgctcttg gcctggttat aaagcttatg ctcatgatca cattgattat cattttgatc 8340
   tttccaaccc gttctattct tttgtaaata cattctacat ttctttaggt gatagaaaag 8400
   aaaaaattta tcttagagtg gttggtgcaa caccaaaaga gaaaagattg aatgttggtt 8460
   gtagaacatc tttctcagtt aacatgccaa ttggaactca gatttaccat gacaaagata 8520
   tgcaatatct tgtcgaggga agacatcttg agtgtgctca cagagtttac tttgtgaagt 8580
   actgtccaaa ccatgcacat ggttactgct ttaatgacag gctaaaggtt tataatcttg 8640
   gtcgtgtcaa aagcagaaag gcttttgaga aaatcaacca acatcagaaa agtgagttgt 8700
   aaggcaaccc gatgtttaaa actggttttt ccgaggaatt actgg 8745
<210> 2
   <211> 4365
   <212> DNA
   <213> Unknown
<220>
   <223> canine coronavirus
<400> 2
   atgattgtga tcttaacttg cgtcttattg ttgtgctcgt accatactgt tgcgagtacg    60
   acaaataatg attgtagaca agttaacgta acacaattag atggcaacga aaatctcatt   120
   agagactttt tgtttcaaaa ctttaaagaa gaaggaactg tagttgttgg tggctattat   180
   cctacagagg tgtggtacaa ctgttctaga acagcaccta ccactgccta tgagtatttt   240
   aataacatac atgcatttta ttttgatatg gaagccatgg aaaatagcac tggtaatgca   300
   cgtggtaaac cgttattgtt tcatgttcat ggtgagcctg ttagtgctat catatatata   360
   tcggcttata gaaatgatgt acaacacagg ccacttttaa aacatgggtt agtgtgcata   420
   actaaaactc gcaatattga ctataacagc ttcactagca gacagtggaa ttccatatgt   480
   acaggtaatg acagaaaaat tcctttctct gtcataccca cggacaatgg aacaaaaatc   540
   tatggacttg agtggaatga cgaatttgtt acagcctata ttagtggtca ttcttataat   600
   tggaacatca ataataattg gtttaacaat gttacactac tttattcccg ctcaagtact   660
   gctacatggc aacacagtgc tgcatatgtt taccaaggtg tttctaactt cacttattac   720
   aagttaaata acaccaatgg tttaaaaact tatgaatttt gtgatgatta tgaatattgc   780
   actggctacg ctactaatgt ctttgctccc actgtaggag gttacatacc tgatggattt   840
   agttttaaca attggttttt gcttacaaat ggctccactt ttgttagtgg cagatttgta   900
   acaaatcaac cattattagt taattgttta tggccagtgc ccagttttgg tgtggcagca   960
   caagaatttt gttttgaagg tgcgcagttt agtcagtgca atggtgtgtc tttaaataac  1020
   acagtggatg ttattagatt caatcttaat tttaccgcag atgtacaatc tggcatgggt  1080
   gctacagtgt tctcattgaa tacaacaggt ggtgtcattc ttgaaatttc atgttataat  1140
   gacacagtga gtgagtctag tttttacagt tatggtgaaa ttccatttgg cataactgat  1200
   ggaccacggt actgttatgc actttacaat ggcacagctc ttaagtattt aggaacatta  1260
   ccacctagtg taaaggaaat tgctattagt aagtggggcc atttttatat taatggttac  1320
   aatttcttta gcacatttcc tattgattgt atatctttta atttaaccac tggtactagt  1380
   ggagcttttt ggactattgc ttacacatcg tacactgaag cattagtaca agttgaaaac  1440
   acagctatta aaaaggtgac gtattgtaac agtcacatta ataacattaa atgttctcaa 1500
   cttactgcta atttgcaaaa tggtttttat cctgttgctt caagtgaagt tggtcttgtc 1560
   aataagagtg ttgtgttact acctagtttc tattcacata ccagtgttaa tataactatt 1620
   gatcttggta tgaagcgtag tggttatggt caacctatag cctcagcatt aagtaacatc 1680
   acactaccga tgcaggataa taacaccgat gtgtattgca ttcgttctaa tcaattttca 1740
   gtttacgtgc attccacttg taaaagttct ttatgggaca atgtgtttaa ttctgactgc 1800
   acagattttt tacatgctac agctgttata aaaactggta cttgtccttt ctcatttgat 1860
   aaattgaaca attacctaac ttttaacaag ttctgttttt cattgaatcc tgttggtgcc 1920
   aattgcaaat ttgatgttgc tgcccgtaca agaaccaatg agcaggttgt tagaagttta 1980
   tatgtgatat atgaagaagg agacaatata gtgggtgtac cgtctgataa tagtggtctg 2040
   cacgatttgt cagtgttaca cttagactcc tgtacagatt acaatatata tggtagaact 2100
   ggtgttggta ttattagaca aactaacagc acactactta gtggcttata ttacacatca 2160
   ctatcaggtg atttgttagg ttttaaaaat gtcactgacg gtgttgtcta ttctgtaaca 2220
   ccatgtgatg taagtgcaca agctgctgtt attgatggtg ccatagttgg agctatgact 2280
   tccattaata gtgaactgtt aggtctaact cattggacaa caacacctaa tttttattat 2340
   tactccatat ataattatac aaatgcgaga actcgtggca ctgcaatcga cagtaacgat 2400
   gttgattgtg aacctatcat aacctattct aatataggtg tttgtaaaaa tggagctttg 2460
   gtttttatta acgtcacaca ttctgatggt gacgttcaac caattagcac cggtaatgtc 2520
   acgataccta caaattttac tatatctgtg caagtcgaat atattcaggt ttacactaca 2580
   ccagtgtcaa tagactgttc aagatacgtc tgcaatggta atcctagatg caataaattg 2640
   ttaacacaat atgtctctgc atgtcaaact attgagcaag cacttgcaat gggtgccaga 2700
   cttgaaaaca tggaagttga ttccatgttg tttgtttctg aaaatgccct taaattggca 2760
   tctgttgaag cattcaatag tacggaaact ctagatccta tttacaaaga atggcctaac 2820
   attggtggtt cttggctagg aggtttaaaa gacatattgc catctcacaa tagcaaacgt 2880
   aagtaccggt ctgctataga agatttgctt tttgataagg ttgtgacatc tggcttaggt 2940
   acagtcgatg aagattacaa acgttgtacg ggtggctatg acatagctga cttagtgtgt 3000
   gcacaatatt acaatggcat catggtgtta cctggtgtgg ctaatgatga caagatggct 3060
   atgtacactg catctcttgc aggtggtata acattaggtg cacttggtgg tggtgcagtg 3120
   tctatacctt ttgcagtagc agttcaggct agacttaatt atgtagcttt acaaactgac 3180
   gtattgaaca aaaaccagca gattctggct aatgctttca atcaagcgat tggtaacatt 3240
   acacaggcat ttggtaaggt taatgatgct atacatcaaa cgtcaaaagg tcttgctact 3300
   gttgctaaag cattggcaaa agtgcaagat gttgttaaca cacaagggca agctttaagc 3360
   cacctaacag tacaattgca aaataatttt caagccatta gtagttccat tagtgacatt 3420
   tataacaggc ttgatgagtt gagtgcggat gcacaagttg acaggctgat tacaggacga 3480
   cttacagcac ttaatgcatt tgtgtctcag actttaacca gacaagcaga ggttagggct 3540
   agtagacaac ttgctaaaga caaagttaat gaatgcgtta ggtctcaatc ccaaagattt 3600
   ggattctgtg gtaatggtac acatttgttt tcacttgcaa atgcagcacc aaatggcatg 3660
   attttctttc acacagtgct attaccaaca gcttatgaaa ctgtgacggc ctggtcaggt 3720
   atttgtgcat cagatggcga tcgcactttt ggacttgttg ttaaagatgt ccagctgacg 3780
   ctatttcgca atttagatga caaattctat ttgacaccta gaactatgta tcagcctaga 3840
   gttgcaacta gttctgattt tgttcaaatt gaagggtgtg atgtcttgtt tgtcaatgca 3900
   actgtaattg agttgcctag tattatacct gattatatcg atattaatca gactgttcaa 3960
   gacatattag aaaattacag accaaattgg acagtacctg aattaacact tgacattttc 4020
   aacgcaacct acttaaacct gactggtgaa attaatgact tagaattcag gtcagaaaag 4080
   ctacataaca ccacggtaga acttgctgtt ctcattgaca atattaacaa tacattagtc 4140
   aatcttgaat ggctcaatag aattgaaact tatgtaaaat ggccttggta tgtatggcta 4200
   ctaataggct tagtagtaat attttgcata ccattactgc tattttgctg ttgtagtaca 4260
   ggttgctgtg ggtgcatagg ttgcttagga agttgttgtc actctatttg tagtagaaga 4320
   caatttgaaa attatgaacc aattgagaaa gtgcatgtcc attaa 4365
<210> 3
   <211> 216
   <212> DNA
   <213> Unknown
<220>
   <223> canine coronavirus
<400> 3
   atggacattg tcaaatctat tgacacatcc gtagacgctg tacttgacga acttgatcgt 60
   gcatactttg ctgtaactct taaagtagag tttaagactg gtaaactact tgtgtgtata 120
   ggttttggtg atacacctct tgaggctaag gataaagcat atgctaaact tggtttctct 180
   attattgaag aagtcaatag tcatacagtt gtttga 216
<210> 4
   <211> 69
   <212> DNA
   <213> Unknown
<220>
   <223> canine coronavirus
<400> 4
   atgctaaact tggtttctct attattgaag aagtcaatag tcatacagtt gtttgatatt      60
   accttttga                                                              69
<210> 5
   <211> 735
   <212> DNA
   <213> Unknown
<220>
   <223> canine coronavirus
<400> 5
   atgattggtg gactttttct taacactctg agttttgtaa ttgttagcaa ccatgtcatt      60
   gttaataata cagcaaatgt gcattctata caacaagaac atgttatagt acaacagcat     120
   caggttgtta gtgctagaac acaaaattac tacccagagt tcagcatcgc tgtactcttt     180
   gtattatttc tagctttgta ccgtagtaca aactttaaga cgtgtgtcga tatcttaatg     240
   tttaagattg tatcaatgac acttataggg cctatgctta tagcatatgg ttactacatt     300
   gatggcattg ttacaacaac tgtcttagct ttaagatttg tctacttatc atacttttgg     360
   tatgttaata gtaggtttga attcatctta tacaatacaa cgacactcat gtttgtacat     420
   ggcagagctg caccgtttat gagaagttct cacagctcta tttatgtcac attgtacggt     480
   ggcataaatt atatgtttgt gaatgacctc acgttgcatt ttgtagaccc tatgcttgta     540
   agcatagcaa tacgtggctt agctcatgct gatctaactg ttgttagagc agttgaactt     600
   ctcaatggtg attttatcta tgtattttca caggagcccg tagtcggtgt ttacaatgca     660
   gccttttctc aggcggttct aaacgaaatt gacttaaaag aagaagaaga agaccatacc     720
   tatgacgttc cctag                                                      735
<210> 6
   <211> 306
   <212> DNA
   <213> Unknown
<220>
   <223> canine coronavirus
<400> 6
   atgctcgttt tcctccatgc tgtatttatc acagttttaa tcttactact aattggtaga      60
   ctccaattat tagaaagatt attacttaat cattccctca atcttaaaac tgtcaataat     120
   gttttaggtg tgactgacac tggtctgaaa gtaaattgct tacagctttt gaaaccagac     180
   tgtcttgatt ttaacatctt acataggagt ttggcagaaa ccagattact aaaagtagta     240
   cttcgagtaa tctttctagt attactaggg ttttgctgct acagattgtt agtcacatta     300
   atttag                                                                306
<210> 7
   <211> 642
   <212> DNA
   <213> Unknown
<220>
   <223> canine coronavirus
<400> 7
   atgaagtttt tgatttttgt actgtgtctt tctcttgtga atggatatgg aattagaaga      60
   agcatacaag aacatgaccc aaaagagtcc catgaacacc cgaccatgac atgggaatta     120
   ttggaaagat ttgttggaag taccttgtac atcacaacaa accaaatcct atctctacca     180
   actggagcgc aaatttattg tgatgagatt gaaggattcc aatgctcttg gcctggttat     240
   aaagcttatg ctcatgatca cattgattat cattttgatc tttccaaccc gttctattct      300
   tttgtaaata cattctacat ttctttaggt gatagaaaag aaaaaattta tcttagagtg     360
   gttggtgcaa caccaaaaga gaaaagattg aatgttggtt gtagaacatc tttctcagtt     420
   aacatgccaa ttggaactca gatttaccat gacaaagata tgcaatatct tgtcgaggga     480
   agacatcttg agtgtgctca cagagtttac tttgtgaagt actgtccaaa ccatgcacat     540
   ggttactgct ttaatgacag gctaaaggtt tataatcttg gtcgtgtcaa aagcagaaag     600
   gcttttgaga aaatcaacca acatcagaaa agtgagttgt aa                        642
<210> 8
   <211> 249
   <212> DNA
   <213> Unknown
<220>
   <223> canine coronavirus
<400> 8
   atgacgttcc ctagggcatt gactgtcata gatgacaatg gaatggtcat tagtatcatt 60
   ttctggttcc tgttgataat tatattgata ttactttcaa tagcattgct aaatataatt 120
   aagctatgca tggtatgttg taacttagga agaacagtta ttattgttcc agcgcaacat 180
   gcctatgatg cctataagaa ttttatgcga attaaagcat ataaccccga cgaagcactc 240
   cttgtttga 249
<210> 9
   <211> 789
   <212> DNA
   <213> Unknown
<220>
   <223> canine coronavirus
<400> 9
   atgaagattt tgttagtatt agcgtgtgca attgcatgcg catgtggtga acgttattgc 60
   gctatgaaat ctgactcaga tacttcgtgt cgcaatggta ccactactga ttgcgaatca 120
   tgcttcaacg gaggtgatct tatttggtat cttgcaaact ggaacttcag ctggtctgta 180
   atattgatcg tttttataac ggttttacaa tatggtagac ctcaatttag ctggttcgtg 240
   tatggcatta aaatgcttat tatgtggcta ctatggccca ttgttctggc tcttacgatt 300
   tttaatgcat actcggaata cgaagtttcc agatatgtaa tgttcggctt tagtgttgca 360
   ggtgcaattg ttacatttat actttggatt atgtattttg ttagatccat tcagttatac 420
   agaaggacta agtcttggtg gtctttcaac cctgaaacta acgcaattct ttgcgttagt 480
   gcattaggaa ggagctatgt gcttcctctt gaaggtgtgc caactggtgt cactttaaca 540
   ttgctctcag ggaatttgta cgctgaaggg ttcaaaattg caggtggtat gaacatcgac 600
   aatttgccaa agtacgtaat ggttgcatta cctagcagga ccattgtcta cacacttgtt 660
   ggtaagaaat tgaaagcaag tagtgcaaca ggatgggctt actatgtaaa atctaaagct 720
   ggtgattact caacagatgc acgaactgac aatttgagtg agcaagaaaa attattacat 780
   atggtataa 789
<210> 10
   <211> 1149
   <212> DNA
   <213> Unknown
<220>
   <223> canine coronavirus
<400> 10
   atggccaacc agggacaacg cgttagctgg ggagatgaat ctaccaaaaa gcgtggtcgt      60
   tccaattctc gtggccggaa gaataatact atacctcttt cattcttcaa ccccattacc     120
   ctccaacaag gttcaaaatt ttggaactta tgtccgagag actttgtacc caaaggaata     180
   ggtaacaagg atcaacagat tggttattgg aatagacaaa ctcgctatcg catggtgaag     240
   ggtcagcgta aagagcttcc tgaaaggtgg ttcttctact atttaggtac tggtcctcac     300
   gccgatgcta aatttaaaga tagaatagat ggagttgtct gggttgccaa ggatggtgcc     360
   atgaataagc caaccacact tggtaatcgt ggtgctaata atgaatccaa agctttgaaa     420
   ttcgatggta aagtaccagt agaatttcaa cttgaagtga accaatcaag ggacaattca     480
   aggtcacgct ctcaatctag atcccagtct agaaatagat ctcaatctag aggaaggcaa     540
   caatccaata acaagaagga tgacagtgta gaacaagctg ttcttgctgc actcaaaaag     600
   ttaggtgttg acacagaaaa acaacaacaa cgctctcgtt ctaaatccaa ggaacgtagc     660
   aactctaaga caagagacac tacacctaag aatgaaaaca aacacacctg gaagagaact     720
   gcaggtaaag gtgatgtgac aaaattttat ggagctagaa gtagttcagc caattttggt     780
   gacagcgatc tcgttgccaa tggtagcggt gccaagcatt acccacaact ggctgaatgt     840
   gttccatctg tatctagcat tctgtttgga agctattgga ctgcaaagga agatggcgac     900
   cagattgaag tcacattcac acataaatac cacttgccaa aagatgatcc taagactgga     960
   caattccttc agcagattaa tgcctatgct cgtccatcag aggtggcaaa agaacagaga    1020
   caacgcaaag ctcgttctaa atctgcagaa aggtcagagc aagaggttgt acctgatgca    1080
   ttaacagaaa attacacaga tgtgtttgat gacacacagg ttgagattat tgatgaggta    1140
   acgaactaa                                                            1149
<210> 11
   <211> 1454
   <212> PRT
   <213> Unknown
<220>
   <223> canine coronavirus
<400> 11
   Met Ile Val Ile Leu Thr Cys Val Leu Leu Leu Cys Ser Tyr His Thr
   1 5 10 15
   Val Ala Ser Thr Thr Asn Asn Asp Cys Arg Gln Val Asn Val Thr Gln
   20 25 30
   Leu Asp Gly Asn Glu Asn Leu Ile Arg Asp Phe Leu Phe Gln Asn Phe
   35 40 45
   Lys Glu Glu Gly Thr Val Val Val Gly Gly Tyr Tyr Pro Thr Glu Val
   50 55 60
   Trp Tyr Asn Cys Ser Arg Thr Ala Pro Thr Thr Ala Tyr Glu Tyr Phe
   65 70 75 80
   Asn Asn Ile His Ala Phe Tyr Phe Asp Met Glu Ala Met Glu Asn Ser
   85 90 95
   Thr Gly Asn Ala Arg Gly Lys Pro Leu Leu Phe His Val His Gly Glu
   100 105 110
   Pro Val Ser Ala Ile Ile Tyr Ile Ser Ala Tyr Arg Asn Asp Val Gln
   115 120 125
   His Arg Pro Leu Leu Lys His Gly Leu Val Cys Ile Thr Lys Thr Arg
   130 135 140
   Asn Ile Asp Tyr Asn Ser Phe Thr Ser Arg Gln Trp Asn Ser Ile Cys
   145 150 155 160
   Thr Gly Asn Asp Arg Lys Ile Pro Phe Ser Val Ile Pro Thr Asp Asn
   165 170 175
   Gly Thr Lys Ile Tyr Gly Leu Glu Trp Asn Asp Glu Phe Val Thr Ala
   180 185 190
   Tyr Ile Ser Gly His Ser Tyr Asn Trp Asn Ile Asn Asn Asn Trp Phe
   195 200 205
   Asn Asn Val Thr Leu Leu Tyr Ser Arg Ser Ser Thr Ala Thr Trp Gln
   210 215 220
   His Ser Ala Ala Tyr Val Tyr Gln Gly Val Ser Asn Phe Thr Tyr Tyr
   225 230 235 240
   Lys Leu Asn Asn Thr Asn Gly Leu Lys Thr Tyr Glu Phe Cys Asp Asp
   245 250 255
   Tyr Glu Tyr Cys Thr Gly Tyr Ala Thr Asn Val Phe Ala Pro Thr Val
   260 265 270
   Gly Gly Tyr Ile Pro Asp Gly Phe Ser Phe Asn Asn Trp Phe Leu Leu
   275 280 285
   Thr Asn Gly Ser Thr Phe Val Ser Gly Arg Phe Val Thr Asn Gln Pro
   290 295 300
   Leu Leu Val Asn Cys Leu Trp Pro Val Pro Ser Phe Gly Val Ala Ala
   305 310 315 320
   Gln Glu Phe Cys Phe Glu Gly Ala Gln Phe Ser Gln Cys Asn Gly Val
   325 330 335
   Ser Leu Asn Asn Thr Val Asp Val Ile Arg Phe Asn Leu Asn Phe Thr
   340 345 350
   Ala Asp Val Gln Ser Gly Met Gly Ala Thr Val Phe Ser Leu Asn Thr
   355 360 365
   Thr Gly Gly Val Ile Leu Glu Ile Ser Cys Tyr Asn Asp Thr Val Ser
   370 375 380
   Glu Ser Ser Phe Tyr Ser Tyr Gly Glu Ile Pro Phe Gly Ile Thr Asp
   385 390 395 400
   Gly Pro Arg Tyr Cys Tyr Ala Leu Tyr Asn Gly Thr Ala Leu Lys Tyr
   405 410 415
   Leu Gly Thr Leu Pro Pro Ser Val Lys Glu Ile Ala Ile Ser Lys Trp
   420 425 430
   Gly His Phe Tyr Ile Asn Gly Tyr Asn Phe Phe Ser Thr Phe Pro Ile
   435 440 445
   Asp Cys Ile Ser Phe Asn Leu Thr Thr Gly Thr Ser Gly Ala Phe Trp
   450 455 460
   Thr Ile Ala Tyr Thr Ser Tyr Thr Glu Ala Leu Val Gln Val Glu Asn
   465 470 475 480
   Thr Ala Ile Lys Lys Val Thr Tyr Cys Asn Ser His Ile Asn Asn Ile
   485 490 495
   Lys Cys Ser Gln Leu Thr Ala Asn Leu Gln Asn Gly Phe Tyr Pro Val
   500 505 510
   Ala Ser Ser Glu Val Gly Leu Val Asn Lys Ser Val Val Leu Leu Pro
   515 520 525
   Ser Phe Tyr Ser His Thr Ser Val Asn Ile Thr Ile Asp Leu Gly Met
   530 535 540
   Lys Arg Ser Gly Tyr Gly Gln Pro Ile Ala Ser Ala Leu Ser Asn Ile
   545 550 555 560
   Thr Leu Pro Met Gln Asp Asn Asn Thr Asp Val Tyr Cys Ile Arg Ser
   565 570 575
   Asn Gln Phe Ser Val Tyr Val His Ser Thr Cys Lys Ser Ser Leu Trp
   580 585 590
   Asp Asn Val Phe Asn Ser Asp Cys Thr Asp Phe Leu His Ala Thr Ala
   595 600 605
   Val Ile Lys Thr Gly Thr Cys Pro Phe Ser Phe Asp Lys Leu Asn Asn
   610 615 620
   Tyr Leu Thr Phe Asn Lys Phe Cys Phe Ser Leu Asn Pro Val Gly Ala
   625 630 635 640
   Asn Cys Lys Phe Asp Val Ala Ala Arg Thr Arg Thr Asn Glu Gln Val
   645 650 655
   Val Arg Ser Leu Tyr Val Ile Tyr Glu Glu Gly Asp Asn Ile Val Gly
               660                 665                 670
   Val Pro Ser Asp Asn Ser Gly Leu His Asp Leu Ser Val Leu His Leu
           675                 680                 685
   Asp Ser Cys Thr Asp Tyr Asn Ile Tyr Gly Arg Thr Gly Val Gly Ile
       690                 695                 700
   Ile Arg Gln Thr Asn Ser Thr Leu Leu Ser Gly Leu Tyr Tyr Thr Ser
   705                 710                 715                 720
   Leu Ser Gly Asp Leu Leu Gly Phe Lys Asn Val Thr Asp Gly Val Val
                   725                 730                 735
   Tyr Ser Val Thr Pro Cys Asp Val Ser Ala Gln Ala Ala Val Ile Asp
               740                 745                 750
   Gly Ala Ile Val Gly Ala Met Thr Ser Ile Asn Ser Glu Leu Leu Gly
           755                 760                 765
   Leu Thr His Trp Thr Thr Thr Pro Asn Phe Tyr Tyr Tyr Ser Ile Tyr
       770                 775                 780
   Asn Tyr Thr Asn Ala Arg Thr Arg Gly Thr Ala Ile Asp Ser Asn Asp
   785                 790                 795                 800
   Val Asp Cys Glu Pro Ile Ile Thr Tyr Ser Asn Ile Gly Val Cys Lys
                   805                 810                 815
   Asn Gly Ala Leu Val Phe Ile Asn Val Thr His Ser Asp Gly Asp Val
               820                 825                 830
   Gln Pro Ile Ser Thr Gly Asn Val Thr Ile Pro Thr Asn Phe Thr Ile
           835                 840                 845
   Ser Val Gln Val Glu Tyr Ile Gln Val Tyr Thr Thr Pro Val Ser Ile
       850                 855                 860
   Asp Cys Ser Arg Tyr Val Cys Asn Gly Asn Pro Arg Cys Asn Lys Leu
   865                 870                 875                 880
   Leu Thr Gln Tyr Val Ser Ala Cys Gln Thr Ile Glu Gln Ala Leu Ala
                   885                 890                 895
   Met Gly Ala Arg Leu Glu Asn Met Glu Val Asp Ser Met Leu Phe Val
               900                 905                 910
   Ser Glu Asn Ala Leu Lys Leu Ala Ser Val Glu Ala Phe Asn Ser Thr
   915 920 925
   Glu Thr Leu Asp Pro Ile Tyr Lys Glu Trp Pro Asn Ile Gly Gly Ser
   930 935 940
   Trp Leu Gly Gly Leu Lys Asp Ile Leu Pro Ser His Asn Ser Lys Arg
   945 950 955 960
   Lys Tyr Arg Ser Ala Ile Glu Asp Leu Leu Phe Asp Lys Val Val Thr
   965 970 975
   Ser Gly Leu Gly Thr Val Asp Glu Asp Tyr Lys Arg Cys Thr Gly Gly
   980 985 990
   Tyr Asp Ile Ala Asp Leu Val Cys Ala Gln Tyr Tyr Asn Gly Ile Met
   995 1000 1005
   Val Leu Pro Gly Val Ala Asn Asp Asp Lys Met Ala Met Tyr Thr
   1010 1015 1020
   Ala Ser Leu Ala Gly Gly Ile Thr Leu Gly Ala Leu Gly Gly Gly
   1025 1030 1035
   Ala Val Ser Ile Pro Phe Ala Val Ala Val Gln Ala Arg Leu Asn
   1040 1045 1050
   Tyr Val Ala Leu Gln Thr Asp Val Leu Asn Lys Asn Gln Gln Ile
   1055 1060 1065
   Leu Ala Asn Ala Phe Asn Gln Ala Ile Gly Asn Ile Thr Gln Ala
   1070 1075 1080
   Phe Gly Lys Val Asn Asp Ala Ile His Gln Thr Ser Lys Gly Leu
   1085 1090 1095
   Ala Thr Val Ala Lys Ala Leu Ala Lys Val Gln Asp Val Val Asn
   1100 1105 1110
   Thr Gln Gly Gln Ala Leu Ser His Leu Thr Val Gln Leu Gln Asn
   1115 1120 1125
   Asn Phe Gln Ala Ile Ser Ser Ser Ile Ser Asp Ile Tyr Asn Arg
   1130 1135 1140
   Leu Asp Glu Leu Ser Ala Asp Ala Gln Val Asp Arg Leu Ile Thr
   1145 1150 1155
   Gly Arg Leu Thr Ala Leu Asn Ala Phe Val Ser Gln Thr Leu Thr
   1160 1165 1170
   Arg Gln Ala Glu Val Arg Ala Ser Arg Gln Leu Ala Lys Asp Lys
   1175 1180 1185
   Val Asn Glu Cys Val Arg Ser Gln Ser Gln Arg Phe Gly Phe Cys
   1190 1195 1200
   Gly Asn Gly Thr His Leu Phe Ser Leu Ala Asn Ala Ala Pro Asn
   1205 1210 1215
   Gly Met Ile Phe Phe His Thr Val Leu Leu Pro Thr Ala Tyr Glu
   1220 1225 1230
   Thr Val Thr Ala Trp Ser Gly Ile Cys Ala Ser Asp Gly Asp Arg
   1235 1240 1245
   Thr Phe Gly Leu Val Val Lys Asp Val Gln Leu Thr Leu Phe Arg
   1250 1255 1260
   Asn Leu Asp Asp Lys Phe Tyr Leu Thr Pro Arg Thr Met Tyr Gln
   1265 1270 1275
   Pro Arg Val Ala Thr Ser Ser Asp Phe Val Gln Ile Glu Gly Cys
   1280 1285 1290
   Asp Val Leu Phe Val Asn Ala Thr Val Ile Glu Leu Pro Ser Ile
   1295 1300 1305
   Ile Pro Asp Tyr Ile Asp Ile Asn Gln Thr Val Gln Asp Ile Leu
   1310 1315 1320
   Glu Asn Tyr Arg Pro Asn Trp Thr Val Pro Glu Leu Thr Leu Asp
   1325 1330 1335
   Ile Phe Asn Ala Thr Tyr Leu Asn Leu Thr Gly Glu Ile Asn Asp
   1340 1345 1350
   Leu Glu Phe Arg Ser Glu Lys Leu His Asn Thr Thr Val Glu Leu
   1355 1360 1365
   Ala Val Leu Ile Asp Asn Ile Asn Asn Thr Leu Val Asn Leu Glu
   1370 1375 1380
   Trp Leu Asn Arg Ile Glu Thr Tyr Val Lys Trp Pro Trp Tyr Val
   1385 1390 1395
   Trp Leu Leu Ile Gly Leu Val Val Ile Phe Cys Ile Pro Leu Leu
   1400 1405 1410
   Leu Phe Cys Cys Cys Ser Thr Gly Cys Cys Gly Cys Ile Gly Cys
   1415 1420 1425
   Leu Gly Ser Cys Cys His Ser Ile Cys Ser Arg Arg Gln Phe Glu
   1430 1435 1440
   Asn Tyr Glu Pro Ile Glu Lys Val His Val His
   1445 1450
<210> 12
   <211> 71
   <212> PRT
   <213> Unknown
<220>
   <223> canine coronavirus
<400> 12
   Met Asp Ile Val Lys Ser Ile Asp Thr Ser Val Asp Ala Val Leu Asp
   1 5 10 15
   Glu Leu Asp Arg Ala Tyr Phe Ala Val Thr Leu Lys Val Glu Phe Lys
   20 25 30
   Thr Gly Lys Leu Leu Val Cys Ile Gly Phe Gly Asp Thr Pro Leu Glu
   35 40 45
   Ala Lys Asp Lys Ala Tyr Ala Lys Leu Gly Phe Ser Ile Ile Glu Glu
   50 55 60
   Val Asn Ser His Thr Val Val
   65 70
<210> 13
   <211> 22
   <212> PRT
   <213> Unknown
<220>
   <223> canine coronavirus
<400> 13
   Met Leu Asn Leu Val Ser Leu Leu Leu Lys Lys Ser Ile Val Ile Gln
   1 5 10 15
   Leu Phe Asp Ile Thr Phe
   20
<210> 14
   <211> 244
   <212> PRT
   <213> Unknown
<220>
   <223> canine coronavirus
<400> 14
   Met Ile Gly Gly Leu Phe Leu Asn Thr Leu Ser Phe Val Ile Val Ser
   1               5                   10                  15
   Asn His Val Ile Val Asn Asn Thr Ala Asn Val His Ser Ile Gln Gln
               20                  25                  30
   Glu His Val Ile Val Gln Gln His Gln Val Val Ser Ala Arg Thr Gln
           35                  40                  45
   Asn Tyr Tyr Pro Glu Phe Ser Ile Ala Val Leu Phe Val Leu Phe Leu
       50                  55                  60
   Ala Leu Tyr Arg Ser Thr Asn Phe Lys Thr Cys Val Asp Ile Leu Met
   65                  70                  75                  80
   Phe Lys Ile Val Ser Met Thr Leu Ile Gly Pro Met Leu Ile Ala Tyr
                   85                  90                  95
   Gly Tyr Tyr Ile Asp Gly Ile Val Thr Thr Thr Val Leu Ala Leu Arg
               100                 105                 110
   Phe Val Tyr Leu Ser Tyr Phe Trp Tyr Val Asn Ser Arg Phe Glu Phe
           115                 120                 125
   Ile Leu Tyr Asn Thr Thr Thr Leu Met Phe Val His Gly Arg Ala Ala
       130                 135                 140
   Pro Phe Met Arg Ser Ser His Ser Ser Ile Tyr Val Thr Leu Tyr Gly
   145                 150                 155                 160
   Gly Ile Asn Tyr Met Phe Val Asn Asp Leu Thr Leu His Phe Val Asp
                   165                 170                 175
   Pro Met Leu Val Ser Ile Ala Ile Arg Gly Leu Ala His Ala Asp Leu
               180                 185                 190
   Thr Val Val Arg Ala Val Glu Leu Leu Asn Gly Asp Phe Ile Tyr Val
           195                 200                 205
   Phe Ser Gln Glu Pro Val Val Gly Val Tyr Asn Ala Ala Phe Ser Gln
   210 215 220
   Ala Val Leu Asn Glu Ile Asp Leu Lys Glu Glu Glu Glu Asp His Thr
   225 230 235 240
   Tyr Asp Val Pro
<210> 15
   <211> 101
   <212> PRT
   <213> unknown
<220>
   <223> canine coronavirus
<400> 15
   Met Leu Val Phe Leu His Ala Val Phe Ile Thr Val Leu Ile Leu Leu
   1 5 10 15
   Leu Ile Gly Arg Leu Gln Leu Leu Glu Arg Leu Leu Leu Asn His Ser
   20 25 30
   Leu Asn Leu Lys Thr Val Asn Asn Val Leu Gly Val Thr Asp Thr Gly
   35 40 45
   Leu Lys Val Asn Cys Leu Gln Leu Leu Lys Pro Asp Cys Leu Asp Phe
   50 55 60
   Asn Ile Leu His Arg Ser Leu Ala Glu Thr Arg Leu Leu Lys Val Val
   65 70 75 80
   Leu Arg Val Ile Phe Leu Val Leu Leu Gly Phe Cys Cys Tyr Arg Leu
   85 90 95
   Leu Val Thr Leu Ile
   100
<210> 16
   <211> 213
   <212> PRT
   <213> Unknown
<220>
   <223> canine coronavirus
<400> 16
   Met Lys Phe Leu Ile Phe Val Leu Cys Leu Ser Leu Val Asn Gly Tyr
   1               5                   10                  15
   Gly Ile Arg Arg Ser Ile Gln Glu His Asp Pro Lys Glu Ser His Glu
               20                  25                  30
   His Pro Thr Met Thr Trp Glu Leu Leu Glu Arg Phe Val Gly Ser Thr
           35                  40                  45
   Leu Tyr Ile Thr Thr Asn Gln Ile Leu Ser Leu Pro Thr Gly Ala Gln
       50                  55                  60
   Ile Tyr Cys Asp Glu Ile Glu Gly Phe Gln Cys Ser Trp Pro Gly Tyr
   65                  70                  75                  80
   Lys Ala Tyr Ala His Asp His Ile Asp Tyr His Phe Asp Leu Ser Asn
                   85                  90                  95
   Pro Phe Tyr Ser Phe Val Asn Thr Phe Tyr Ile Ser Leu Gly Asp Arg
               100                 105                 110
   Lys Glu Lys Ile Tyr Leu Arg Val Val Gly Ala Thr Pro Lys Glu Lys
           115                 120                 125
   Arg Leu Asn Val Gly Cys Arg Thr Ser Phe Ser Val Asn Met Pro Ile
       130                 135                 140
   Gly Thr Gln Ile Tyr His Asp Lys Asp Met Gln Tyr Leu Val Glu Gly
   145                 150                 155                 160
   Arg His Leu Glu Cys Ala His Arg Val Tyr Phe Val Lys Tyr Cys Pro
                   165                 170                 175
   Asn His Ala His Gly Tyr Cys Phe Asn Asp Arg Leu Lys Val Tyr Asn
               180                 185                 190
   Leu Gly Arg Val Lys Ser Arg Lys Ala Phe Glu Lys Ile Asn Gln His
           195                 200                 205
   Gln Lys Ser Glu Leu
       210
<210> 17
   <211> 82
   <212> PRT
   <213> Unknown
<220>
   <223> canine coronavirus
<400> 17
   Met Thr Phe Pro Arg Ala Leu Thr Val Ile Asp Asp Asn Gly Met Val
   1               5                   10                  15
   Ile Ser Ile Ile Phe Trp Phe Leu Leu Ile Ile Ile Leu Ile Leu Leu
               20                  25                  30
   Ser Ile Ala Leu Leu Asn Ile Ile Lys Leu Cys Met Val Cys Cys Asn
           35                  40                  45
   Leu Gly Arg Thr Val Ile Ile Val Pro Ala Gln His Ala Tyr Asp Ala
       50                  55                  60
   Tyr Lys Asn Phe Met Arg Ile Lys Ala Tyr Asn Pro Asp Glu Ala Leu
   65                  70                  75                  80
   Leu Val
<210> 18
   <211> 382
   <212> PRT
   <213> Unknown
<220>
   <223> canine coronavirus
<400> 18
   Met Ala Asn Gln Gly Gln Arg Val Ser Trp Gly Asp Glu Ser Thr Lys
   1               5                   10                  15
   Lys Arg Gly Arg Ser Asn Ser Arg Gly Arg Lys Asn Asn Thr Ile Pro
               20                  25                  30
   Leu Ser Phe Phe Asn Pro Ile Thr Leu Gln Gln Gly Ser Lys Phe Trp
           35                  40                  45
   Asn Leu Cys Pro Arg Asp Phe Val Pro Lys Gly Ile Gly Asn Lys Asp
       50                  55                  60
   Gln Gln Ile Gly Tyr Trp Asn Arg Gln Thr Arg Tyr Arg Met Val Lys
   65                  70                  75                  80
   Gly Gln Arg Lys Glu Leu Pro Glu Arg Trp Phe Phe Tyr Tyr Leu Gly
                   85                  90                  95
   Thr Gly Pro His Ala Asp Ala Lys Phe Lys Asp Arg Ile Asp Gly Val
               100                 105                 110
   Val Trp Val Ala Lys Asp Gly Ala Met Asn Lys Pro Thr Thr Leu Gly
   115 120 125
   Asn Arg Gly Ala Asn Asn Glu Ser Lys Ala Leu Lys Phe Asp Gly Lys
   130 135 140
   Val Pro Val Glu Phe Gln Leu Glu Val Asn Gln Ser Arg Asp Asn Ser
   145 150 155 160
   Arg Ser Arg Ser Gln Ser Arg Ser Gln Ser Arg Asn Arg Ser Gln Ser
   165 170 175
   Arg Gly Arg Gln Gln Ser Asn Asn Lys Lys Asp Asp Ser Val Glu Gln
   180 185 190
   Ala Val Leu Ala Ala Leu Lys Lys Leu Gly Val Asp Thr Glu Lys Gln
   195 200 205
   Gln Gln Arg Ser Arg Ser Lys Ser Lys Glu Arg Ser Asn Ser Lys Thr
   210 215 220
   Arg Asp Thr Thr Pro Lys Asn Glu Asn Lys His Thr Trp Lys Arg Thr
   225 230 235 240
   Ala Gly Lys Gly Asp Val Thr Lys Phe Tyr Gly Ala Arg Ser Ser Ser
   245 250 255
   Ala Asn Phe Gly Asp Ser Asp Leu Val Ala Asn Gly Ser Gly Ala Lys
   260 265 270
   His Tyr Pro Gln Leu Ala Glu Cys Val Pro Ser Val Ser Ser Ile Leu
   275 280 285
   Phe Gly Ser Tyr Trp Thr Ala Lys Glu Asp Gly Asp Gln Ile Glu Val
   290 295 300
   Thr Phe Thr His Lys Tyr His Leu Pro Lys Asp Asp Pro Lys Thr Gly
   305 310 315 320
   Gln Phe Leu Gln Gln Ile Asn Ala Tyr Ala Arg Pro Ser Glu Val Ala
   325 330 335
   Lys Glu Gln Arg Gln Arg Lys Ala Arg Ser Lys Ser Ala Glu Arg Ser
   340 345 350
   Glu Gln Glu Val Val Pro Asp Ala Leu Thr Glu Asn Tyr Thr Asp Val
   355 360 365
   Phe Asp Asp Thr Gln Val Glu Ile Ile Asp Glu Val Thr Asn
   370 375 380
<210> 19
   <211> 262
   <212> PRT
   <213> Unknown
<220>
   <223> canine coronavirus
<400> 19
   Met Lys Ile Leu Leu Val Leu Ala Cys Ala Ile Ala Cys Ala Cys Gly
   1               5                   10                  15
   Glu Arg Tyr Cys Ala Met Lys Ser Asp Ser Asp Thr Ser Cys Arg Asn
               20                  25                  30
   Gly Thr Thr Thr Asp Cys Glu Ser Cys Phe Asn Gly Gly Asp Leu Ile
           35                  40                  45
   Trp Tyr Leu Ala Asn Trp Asn Phe Ser Trp Ser Val Ile Leu Ile Val
       50                  55                  60
   Phe Ile Thr Val Leu Gln Tyr Gly Arg Pro Gln Phe Ser Trp Phe Val
   65                  70                  75                  80
   Tyr Gly Ile Lys Met Leu Ile Met Trp Leu Leu Trp Pro Ile Val Leu
                   85                  90                  95
   Ala Leu Thr Ile Phe Asn Ala Tyr Ser Glu Tyr Glu Val Ser Arg Tyr
               100                 105                 110
   Val Met Phe Gly Phe Ser Val Ala Gly Ala Ile Val Thr Phe Ile Leu
           115                 120                 125
   Trp Ile Met Tyr Phe Val Arg Ser Ile Gln Leu Tyr Arg Arg Thr Lys
       130                 135                 140
   Ser Trp Trp Ser Phe Asn Pro Glu Thr Asn Ala Ile Leu Cys Val Ser
   145                 150                 155                 160
   Ala Leu Gly Arg Ser Tyr Val Leu Pro Leu Glu Gly Val Pro Thr Gly
                   165                 170                 175
   Val Thr Leu Thr Leu Leu Ser Gly Asn Leu Tyr Ala Glu Gly Phe Lys
               180                 185                 190
   Ile Ala Gly Gly Met Asn Ile Asp Asn Leu Pro Lys Tyr Val Met Val
           195                 200                 205
   Ala Leu Pro Ser Arg Thr Ile Val Tyr Thr Leu Val Gly Lys Lys Leu
       210                 215                 220
   Lys Ala Ser Ser Ala Thr Gly Trp Ala Tyr Tyr Val Lys Ser Lys Ala
   225                 230                 235                 240
   Gly Asp Tyr Ser Thr Asp Ala Arg Thr Asp Asn Leu Ser Glu Gln Glu
                   245                 250                 255
   Lys Leu Leu His Met Val
               260

## Description

The present invention relates generally to canine coronavirus (CCoV) infections, and specifically to a pantropic, highly pathogenic variant of CCoV type II that is responsible for acute, fatal disease in dogs. The invention also provides polynucleotides and proteins isolated from the canine coronavirus, and their use in the prophylaxis, therapy and diagnosis of canine infections.

### Background of the invention

Coronaviruses are large enveloped positive-stranded RNA viruses (1). Three different coronaviruses have been identified in dogs thus far (2,3). Canine coronavirus (CCoV) type I and type II are included in group 1 coronaviruses and their evolution is tightly intermingled with that of feline coronaviruses (FCoV type I and type II). FCoV type II has originated by heterologous recombination between CCoV type II and FCoV type I, while CCoV type I is genetically more similar to FCoV type I than to CCoV type II (3). In addition, two FCoV biotypes, that differ in their pathogenicity, have been observed in cats and the onset of acute fatal forms of diseases (referred to as feline infectious peritonitis) is accounted for by the onset of pantropic variants (able to disseminate throughout the organism) of enteric FCoVs, that are likely related to deletions/recombinations in the 3c and 7b genes at the 3' end of FCoV genome (4). Similarly, drastic shift of tissue tropisms in porcine and murine coronaviruses (5,6) and adaptation to humans of the recently-recognized SARS-associated coronavirus (SARS-CoV) (7) has been related to even minimal genome mutations and/or deletions. A third canine coronavirus, CRCoV, detected in the respiratory tract, shares up to 96.0% amino acid (aa) conservation in the spike protein S with bovine coronavirus, within group 2 coronaviruses, providing strong evidence for a recent host-species shift (2).

Coronavirus infection in dogs is usually restricted to the enteric tract. The infection is self-limiting and in general produces only mild or even asymptomatic forms of enteritis (8).

### Description of the invention

A highly pathogenic pantropic variant of canine coronavirus has been isolated from dogs presenting severe internal lesions. Experimental infection of dogs with the virus isolate resulted in a severe systemic disease that mimicked the clinical signs originally observed in the animals. The sequence of the 3' genome end of the pantropic CCoV strain was determined by RT-PCR amplification and sequencing of overlapping fragments. The structural and nonstructural proteins of the newly isolated CCoV strain displayed high sequence identity to those of other type II CCoVs, with the exceptions of nonstructural proteins (nsp) 3b and 3c, the sequences of which resulted significantly different in the isolated strain and in known type II canine coronaviruses, respectively.

In one aspect the present invention provides the partial genomic sequence of the newly isolated coronavirus strain (SEQ ID NO: 1), which can be used to prepare diagnostic reagents or tools for virus detection in dogs. Specifically, the nucleotide fragments of SEQ ID NO: 1, or sequences complementary thereto, can be used as diagnostic probes or PCR primers in a diagnostic method employing the polymerase chain reaction (PCR) technique to identify the presence of the virus in animal body fluids or tissues, e.g. for the screening of sera. In a typical application, tissue samples from a dog suspected to have come into contact with the coronavirus strain are subjected to PCR amplification using virus-specific primers. The latter are preferably selected from virus-specific genomic regions that allow to distinguish the coronavirus strain herein disclosed. The most distinctive and virus-specific genomic regions from which suitable PCR primers may be selected are those containing the sequences coding for the spike protein (S) (SEQ ID NO: 2), the non-structural proteins 3a, 3b, 3c, 7a and 7b (SEQ ID NO: 3, 4, 5, 6 and 7, respectively), the envelope protein E (SEQ ID NO: 8), the membrane protein M (SEQ ID NO: 9) and the nucleocapsid protein N (SEQ ID NO: 10). Particularly preferred are the genomic regions containing the sequence coding for the non-structural proteins 3b and 3c, which show significant alterations in the isolated coronavirus strain compared to phylogenetically-related viruses.

In a particularly preferred embodiment, the diagnostic primers or probes are designed to identify the 38-nucleotide deletion in the nsp3b gene.

Virus-specific proteins, preferably the non-structural proteins nsp3b and nsp3c, and antigenic peptides thereof, can be used to assemble immuno- or radioimmuno assays, such as ELISA or Western blot, or for the development of antibodies. The preferred antigens for use in immunoassays and for developing antibodies are selected from the structural and non-structural proteins herein provided, the sequences of which are listed in SEQ ID NO: 11 (spike protein S), SEQ ID NO: 12 (nsp3a), SEQ ID NO: 13 (nsp3b), SEQ ID NO: 14 (nsp3c), SEQ ID NO: 15 (nsp7a), SEQ ID NO: 16 (nsp7b), SEQ ID NO: 17 (envelope protein E), SEQ ID NO: 18 (nucleocapsid protein N), SEQ ID NO: 19 (membrane protein M). The virus-specific sequences of nsp3b and nsp3c (SEQ ID NOs: 13 and 14) identify preferred antigens for use in a diagnostic method according to the invention.

In a further aspect, the invention provides antibodies to one or more epitopes in the above identified amino acid sequences. The antibodies may be monoclonal, polyclonal or fragments such as Fab, Fv and scFv.

In a yet further embodiment, the invention provides a vaccine composition containing an immunogenic protein, which is preferably selected from SEQ ID NO: 11 through SEQ ID NO: 19, or an avirulent antigenic product obtained by attenuation or inactivation of the coronavirus pathogenic strain herein disclosed. The latter can be isolated from organs or tissues of infected animals, propagated in mammalian cells, recovered and concentrated to an appropriate viral titer, inactivated by chemical treatment or attenuated by repeated passages in suitable cells, especially in feline or canine cells. The coronavirus strain can be easily identified by detection of specific molecular markers, such as proteins nsp 3b and 3c (SEQ ID NOs: 13 and 14). The preparation of a vaccine composition containing, in addition to the active ingredient, appropriate vehicles, excipients or adjuvants, is within the skill of the art. The vaccine composition may be used for the prohylaxis or treatment of infections caused by the pantropic, highly-virulent CCoV strain in dogs, particularly in young animals and pups.

### Detailed description of the invention

Recently, a severe outbreak of a fatal systemic disease occurred in a pet shop in Bari, Italy. Clinical signs were observed initially in three Miniature Pinscher and in a Spaniel Cocker, 45 and 53 days of age, respectively, and consisted of fever (39.5-40°C), lethargy, inappetance, vomiting, hemorrhagic diarrhea, and neurological signs (ataxia, seizures) with death after 2 days. After few days, the same signs were observed in two additional 45-day-old Miniature Pinschers and in a 56-day-old Pekinese dog. At necropsy, the dogs showed hemorrhagic enteritis, abundant serosanguineous fluid in the abdominal cavity and severe lesions in the parenchymatous organs. The lungs had multiple, patchy, red areas of consolidation. The liver was yellowish-brown and congested, with hemorrhages on its surface, while the spleen was enlarged with subcapsular hemorrhages. Variable gross changes in other organs included multifocal hemorrhagic renal cortical infarcts and petechial hemorrhages on the lymph node surface.

Virological and bacteriological investigations on the parenchymatous organs failed to detect common canine pathogens, notably canine parvovirus type-2, canine distemper virus, canine adenovirus type-1 and type-2, whereas CCoV type I and type II were identified in the intestinal content of all pups by genotype-specific real-time RT-PCR assays (9). Unexpectedly, CCoV type II RNA was also detected in lungs (median titers of 1.08×10⁶ RNA copies/µl of template), spleen (median titers of 4.46×10⁶ RNA copies/µl of template), liver (median titers of 9.02 × 10⁴ RNA copies/µl of template), kidney (median titers of 7.54×10⁴ RNA copies/µl of template), and brain (median titers of 5.23×10³ RNA copies/µl of template). Virus-induced cytopthatic effect was observed in A-72 cells and CCoV type II strain (CB/05) was isolated from all the examined tissues but brain.

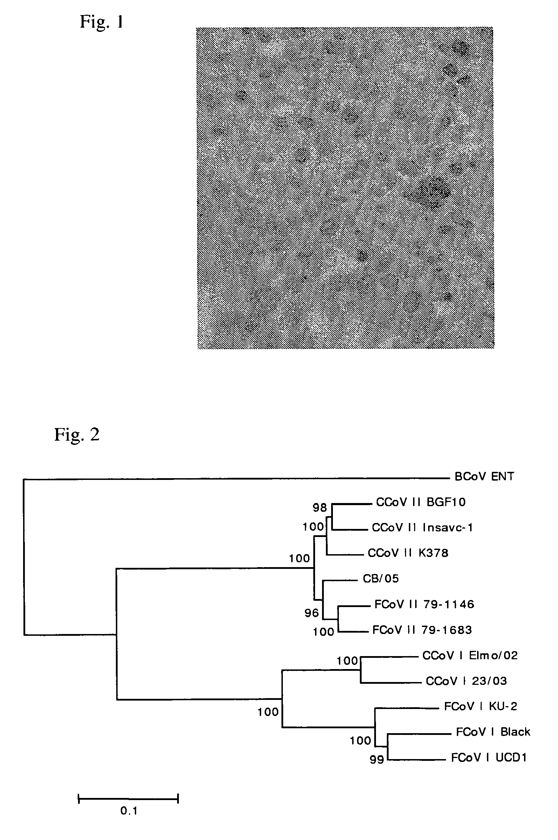

Immunohistochemistry using a CCoV-specific monoclonal antibody detected CCoV antigen in the organs with gross lesions that were examined (lungs, kidneys, liver, spleen, gut and lymph nodes (Fig. 1).

The sequence of the 3' genome end (8.8 kb) of the pantropic CCoV strain was determined by RT-PCR amplification and sequencing of overlapping fragments. The structural proteins S, E, M, N displayed a high degree of aa identity to the cognate ORFs of CCoV type II, The S protein of strain CB/05 displayed the highest identity to FCoV type II strain 79-1146 (Fig. 2). Due to a lack of data from the 3' end of the CCoV genome in the genes encoding for nonstructural proteins (3a, 3b, 3c, 7a and 7b), comparison of strain CB/05 was possible only with CCoV type II strains Insavc-1 (10) and BGF (11) and with CCoV type I strains Elmo/02 and 23/03 (3,12). The nonstructural proteins (nsp) 3a, 7a and 7b presented slight sequence-alterations. The nsp3b was 49-aa shorter (22 aa) than expected due to the presence of a 38-nt deletion and to a frame shift in the sequence downstream the deletion that introduced an early stop codon. The nsp3c (244 aa) was 6-aa shorter and 79-aa longer than the cognate proteins of the enteropathogen strain BGF and of the attenuated strain Insavc-1a, respectively.

To confirm the pathogenic potential of strain CB/05, two 6-month-old dogs were infected experimentally (authorization no. 67/2002-C released by Ministry of Health of Italy). Two ml of the cryolysate of a lung-derived 1^{st} virus passage in A-72 cells were administered intranasally to the dogs. The cell cryolysate tested negative to other common canine pathogens and displayed an infectivity dose of 10^{5.50} TCID₅₀/50 µl on A-72 cells, and a titer of 1.18×10⁷ RNA copies/µl of template by real-time RT-PCR. The virus was re-isolated from the experimentally-infected dogs. Severe clinical signs, characterized by pyrexia (39.8-40.1°C), anorexia, depression, vomiting, diarrhea and leukopenia, were observed, that persisted 8-10 days. Despite the severe symptoms, the dogs slowly recovered from illness.

### Description of the Figures

**Figure 1****.** Immunohistochemistry on a section of lung tissue. CCoV antigen detection (brown staining) by monoclonal antibody. 400X.
**Figure 2****.** Neighbor-joining tree of the S protein of canine and feline coronaviruses. For the phylogenetic analysis the following reference strains were used: CCoV type I strains Elmo/02 (accession no AY307020) and 23/03 (AY307021); CCoV type II strains Insavc-1 (D13096) and K378 (X77047); FCoV type I strains KU-2 (D32044), Black (AB088223) and UCD-1 (AB088222); FCoV type II strains 79-1146 (X06170) and 79-1683 (X80799); bovine coronavirus (BCoV) strain ENT (NC_003045). The tree is rooted on BCoV-ENT and drawn to scale. A statistical support was provided by bootstrapping over 100 replicates.

### References

1. Lai MMC, Holmes KV. Coronaviridae: The viruses and their replication. In: Knipe DM, Howley PM, editors. Fields Virology, 4th edition. Philadelphia, PA: Lippincott Williams and Wilkins. 2001; p.1163-85.
2. Erles K, Toomey C, Brooks HW, Brownlie. J. Detection of a group 2 coronavirus in dogs with canine infectious respiratory disease. Virology. 2003;310:216-23.
3. Pratelli A, Martella V, Decaro N, Tinelli A, Camero M, Cirone F, et al. Genetic diversity of a canine coronavirus detected in pups with diarrhoea in Italy. J Virol Methods. 2003; 110:9-17.
4. Vennema H, Poland A, Foley J, Pedersen NC. Feline infectious peritonitis viruses arise by mutation from endemic feline enteric coronaviruses. Virology. 1998;243:150-7.
5. Laude H, Van Reeth K, Pensaert M. Porcine respiratory coronavirus: molecular features and virus-host interactions. Vet Res. 1993;24:125-50.
6. Haspel MV, Lampert PW, Oldstone MB. Temperature-sensitive mutants of mouse hepatitis virus produce a high incidence of demyelination. Proc Natl Acad Sci USA. 1978;75:4033-36.
7. Guan Y, Zheng BJ, He YQ, Liu XL, Zhuang ZX, Cheung CL, et al. Isolation and characterization of viruses related to the SARS coronavirus from animals in southern China. Science, 2003;302:276-8.
8. Tennant BJ, Gaskell RM, Kelly DF, Carter SD, Gaskell CJ. Canine coronavirus infection in the dog following oronasal inoculation. Res Vet Sci. 1991;51:11-8.
9. Decaro N, Martella V, Ricci D, Elia G, Desario C, Campolo M, et al. Genotype-specific fluorogenic RT-PCR assays for the detection and quantitation of canine coronavirus type I and type II RNA in faecal samples of dogs. J Virol Methods, in press.
10. Horsburgh BC, Brierley I, Brown TD. Analysis of a 9.6 kb sequence from the 3' end of canine coronavirus genomic RNA. J Gen Virol. 1992;73:2849-62.
11. Sanchez-Morgado JM, Poynter S, Morris TH. Molecular characterization of a virulent canine coronavirus BGF strain. Virus Res. 2004;104:27-31.
12. Pratelli A, Decaro N, Tinelli A, Martella V, Elia G, Tempesta M, et al, Two genotypes of canine coronavirus simultaneously detected in fecal samples of dogs with diarrhea. J Clin Microbiol. 2004;42:1797-9.
13. Jonassen CM, Kofstad T, Larsen IL, Lovland A, Handeland K, Follestad A, et al, Molecular identification and characterization of novel coronaviruses infecting graylag geese (Anser anser), feral pigeons (Columbia livia) and mallards (Anas platyrhynchos). J Gen Virol. 2005;86:1597-607

## Claims

1. An isolated canine coronavirus, wherein said virus:
i) is a pantropic variant of CCoV type II and can be detected in the lungs, spleen, liver, kidneys or brain of an infected dog;
ii) has as a part of its genome the sequence represented by SEQ ID NO:1; and
iii) contains the polynucleotide sequences coding for the spike protein (S) (SEQ ID NO: 2), the non-structural proteins 3a, 3b, 3c, 7a and 7b (SEQ ID NO: 3, 4, 5, 6 and 7, respectively), the envelope protein E (SEQ ID NO: 8), the membrane protein M (SEQ ID NO: 9) and the nucleocapsid protein N (SEQ ID NO: 10).

2. A coronavirus according to claim 1, which is inactivated or attenuated.

3. A polynucleotide isolated from the virus of claims 1-2, wherein said polynucleotide consists of SEQ ID NO:4.

4. A polynucleotide isolated from the virus of claims 1-2, wherein said polynucleotide consists of SEQ ID NO:5.

5. An isolated CCoV protein that consists of the polypeptide encoded by the polynucleotide of claim 3 or 4.

6. A CCoV protein according to claim 5, wherein said protein is selected from the group consisting of SEQ ID NO:13 and SEQ ID NO:14.

7. The use of a polynucleotide according to claims 3-4 or of a protein according to claims 5-6 in an *in-vitro* biological assay for CCoV detection in a dog fluid or tissue sample.

8. The use according to claim 7, wherein the *in vitro* biological assay is one of the following: ELISA, Western blot, (RT)PCR.

9. The use of a CCoV protein according to claim 5 or 6, to prepare an immunogenic composition for use in the generation of antibodies in animals.

10. A vaccine composition containing, as an immunogenic component, an attenuated or inactivated coronavirus according to claim 2, together with pharmaceutically acceptable vehicles and excipients.

11. The vaccine composition according to claim 10, for use in the prophylaxis or treatment of infections caused by pantropic virulent CCoV in dogs.

## Patentansprüche

1. Isolierter Hundecoronavirus, worin der Virus:
i) eine pantropische Variante von CCoV Typ II ist und in der Lunge, Milz, Leber, den Nieren oder dem Gehirn eines infizierten Hundes nachgewiesen werden kann;
ii) als einen Teil seines Genoms die durch SEQ ID Nr. 1 dargestellte Sequenz aufweist; und
iii) die Polynukleotidsequenzen enthält, welche für das Spike-Protein (S) (SEQ ID Nr. 2), die nicht-strukturellen Proteine 3a, 3b, 3c, 7a und 7b (SEQ ID Nr. 3, 4, 5, 6 beziehungsweise 7), das Hüllprotein E (SEQ ID Nr. 8), das Membranprotein M (SEQ ID Nr. 9) und das Nucleocapsid-Protein N (SEQ ID Nr. 10) kodieren.

2. Coronavirus nach Anspruch 1, der inaktiviert oder abgeschwächt ist.

3. Polynukleotid, welches aus dem Virus gemäß den Ansprüchen 1 bis 2 isoliert ist, worin das Polynukleotid aus SEQ ID Nr. 4 besteht.

4. Polynukleotid, welches aus dem Virus gemäß den Ansprüchen 1 bis 2 isoliert ist, worin das Polynukleotid aus SEQ ID Nr. 5 besteht.

5. Isoliertes CCoV-Protein, welches aus dem durch das Polynukleotid gemäß des Anspruchs 3 oder 4 kodierten Polypeptid besteht.

6. CCoV-Protein nach Anspruch 5, worin das Protein ausgewählt ist aus der Gruppe, bestehend aus SEQ ID Nr. 13 und SEQ ID Nr. 14.

7. Verwendung eines Polynukleotids nach den Ansprüchen 3 bis 4 oder eines Proteins nach den Ansprüchen 5 bis 6 in einem biologischen *in-vitro* Test zum Nachweis von CCoV in einer Fluid- oder einer Gewebeprobe eines Hundes.

8. Verwendung nach Anspruch 7, wobei der biologische *in-vitro* Test einer der Folgenden ist: ELISA, Western Blot, (RT)PCR.

9. Verwendung eines CCoV-Proteins nach Anspruch 5 oder 6, um eine eine Immunreaktion auslösende Zusammensetzung, zur Verwendung zur Antikörperbildung in Tieren, herzustellen.

10. Impfstoff-Zusammensetzung, enthaltend einen abgeschwächten oder inaktivierten Coronavirus nach Anspruch 2, als eine Immunreaktion auslösende Komponente, zusammen mit pharmazeutisch annehmbaren Vehikeln und Arzneimittelträgern.

11. Impfstoff-Zusammensetzung nach Anspruch 10, zur Verwendung in der Prophylaxe oder zur Behandlung von durch pantropischen, virulenten CCoV in Hunden verursachten Infektionen.

## Revendications

1. Coronavirus canin isolé, où ledit virus :
i. est un variant pantropique de CCoV de type II et peut être détecté dans les poumons, la rate, le foie, les reins ou le cerveau d'un chien infectés
ii. a comme partie de son génome la séquence représentée par SEQ ID NO:1; et
iii. contient les séquences polynucléotidiques codant pour la protéine de spicule (S) (SEQ ID NO : 2), les protéines non structurales 3a, 3b, 3c, 7a et 7b (SEQ ID NO : 3, 4, 5, 6, 7, respectivement), la protéine d'enveloppe E (SEQ ID NO : 8), la protéine de membrane M (SEQ ID NO : 9) et la protéine de nucléocapside N (SEQ ID NO : 10).

2. Coronavirus selon la revendication 1, qui est inactivé ou atténué.

3. Polynucléotide isolé à partir du virus des revendications 1 à 2, dans lequel ledit polynucléotide est constitué de SEQ ID NO : 4.

4. Polynucléotide isolé à partir du virus des revendications 1 à 2, dans lequel ledit polynucléotide est constitué de SEQ ID NO : 5.

5. Protéine de CCoV isolée qui est constituée du polypeptide codé par le polynucléotide de la revendication 3 ou 4.

6. Protéine de CCoV selon la revendication 5, où ladite protéine est choisie dans le groupe constitué de SEQ ID NO : 13 et SEQ ID NO : 14.

7. Utilisation d'un polynucléotide selon les revendications 3 à 4 ou d'une protéine selon les revendications 5 à 6 dans un essai biologique *in vitro* pour détecter CCoV dans un échantillon de liquide ou de tissu de chien.

8. Utilisation selon la revendication 7, dans laquelle l'essai biologique *in vitro* est l'un des suivants : ELISA, Western blot, (RT)PCR.

9. Utilisation d'une protéine CCoV selon la revendication 5 ou 6, pour préparer une composition immunogène pour une utilisation dans la génération d'anticorps chez les animaux.

10. Composition vaccinale contenant, en tant que composant immunogène, un coronavirus inactivé ou atténué selon la revendication 2, associé à des véhicules pharmaceutiquement acceptables et des excipients.

11. Composition vaccinale selon la revendication 10, pour une utilisation dans la prophylaxie ou le traitement d'infections causées par un CCoV virulent pantropique chez les chiens.
